# EUROPEAN PATENT APPLICATION

(11) **EP 2 191 787 A1**
(43) Date of publication of application: **02.06.2010**
(21) Application number: 10153261.2
(22) Date of filing: 27.06.2007
(51) Int. Cl.: A61C 8/00

(54) **Drill tool for lifting bone membrane**

(62) Divisional of application: 07763799.9
(71) Applicant: Dricot, Roland, 1703 Dilbeek (BE)
(72) Inventor: Dricot, Roland, 1703 Dilbeek (BE)
(74) Representative: Bird, Ariane

(57) **Abstract**

Tool for creating a perforation or cavity in a bone or bone structure in contact with a membrane, said tool comprising:
- drill having a free end and a channel adapted for supplying a liquid under pressure to the free end;
- means for providing said liquid under pressure such that said liquid flows partially into and through the bone or bone structure toward the membrane while piercing the bone or bone structure with the drill, allowing to perform a progressive detachment of the membrane when the drill is close to the membrane while still being inside the bone or bone structure.

## Description

The subject of the present invention is a structure capable of promoting the formation of bone around an implant, the latter advantageously comprising a body, advantageously made of titanium, having a portion with a screw thread adapted to cooperate with a bone structure so as to provide a primary fixation of the implant relative to the bone.

There is an implant known from the document WO2006/089380 comprising an osteogenic coating comprising a matrix or a binder and particles of calcium sulfate. The coating has a microporosity formed of pores or channels with a diameter of between 20 and 70µ. The coating is in the form of a gel and may include hydroxyapatite.

It has now been observed that by using a structure that is independent of the implant and has a cavity of a particular shape, it is possible to obtain excellent and fast osteointegration, particularly in the lower or upper maxilla.

By using a particular structure having a particular cavity for receiving a part of the implant, it is possible to obtain good control over the quality of the structure, both on the outside and on the inside (the surface of the cavity). The practitioner needing to use the structure can thus control the quality of the structure and that of the implant separately. In addition, the implant must often undergo sterilization treatments that are unsuitable or that can damage the structure promoting the formation of bone.

Moreover, by machining the cavity (mechanically, for example with a drill or drills), an inside surface having pores that are well adapted to bone cell colonization is obtained

The structure according to the invention is a structure for promoting the formation of bone around an implant advantageously made of titanium, said structure being adapted for being at least partly inserted into a bone or a living bone structure or for being in contact with or in proximity to a bone or a living bone structure, said structure comprising an osteogenic body made from a bone reconstruction material essentially comprising pharmaceutical grade calcium sulfate and a slowly or non-resorbable calcium phosphate or apatite biomaterial in the form of porous granules having an average grain size by weight of between 250 and 3000µm, or advantageously between 300µm and 1000µm, said biomaterial granules themselves having a specific surface area of between 10 and 50m²/g, a macroporosity formed of pores or surface cells with an average diameter of between 2µm and 100µm, advantageously between 20µm and 100µm, and preferably between 50µm and 100µm, said osteogenic body having a substantially flat end with an opening extended by a central cavity with a central axis and adapted for receiving at least one end of an implant, said cavity having at least two successive substantially cylindrical parts, i.e. at least a first substantially cylindrical part with a first diameter adjacent to the opening and a second substantially cylindrical part with a second diameter smaller than said first diameter, said successive substantially cylindrical parts being joined to each other by an intermediate zone forming a ledge or a step extending between a substantially circular part of an edge of a first substantially cylindrical part and a substantially circular part of an edge of the second substantially cylindrical part, said substantially circular parts of said edges of said substantially cylindrical parts being separated from one another by a distance measured perpendicular to the central axis of the cavity of between 0.03 times and 0.25 times the diameter of the first substantially cylindrical part, the ratio by weight of calcium sulfate calculated in dihydrate form to porous calcium phosphate or apatite granules in dry form being between 1:3 and 3:1.

Advantageously, the biomaterial in dry form comprises, and is preferably composed of more than 50% by weight (more specifically more than 75% by weight, and for example more than 90% by weight) of fluorohydroxyapatite agglomerates.

According to one embodiment, the biomaterial includes calcium carbonate and hydroxyapatite or carbonated hydroxyapatite, the ratio by weight of calcium carbonate to hydroxyapatite of the biomaterial being between 1.5:100 and 5:100, or advantageously between 2:100 and 3.5:100.

In a preferred embodiment, the biomaterial includes an organic phase, the organic phase representing between 1% by weight and 5% by weight of the biomaterial in dry form. This organic phase is advantageously composed of collagen and/or fats and/or protein.

The biomaterial is preferably a hydroxyapatite derived from algae. The biomaterial is for example prepared by hydrothermal conversion of the calcium carbonate of an alga in the presence of ammonium phosphate at a high temperature (more than 500°C, for example 600°C, 700°C, or 800°C). The process is conducted so as to obtain granules, or agglomerations of elementary particles, granules having good microscopic and macroscopic porosity. In the present specification, the term macroscopic pores is also intended to mean cavities, asperities or cells formed on the surface. The cells or pores are advantageously at least partially covered with microbubbles promoting the attachment of bone cells and the colonization of the structure.

According to one feature, the structure has an end part into which the bottom of the cavity extends. This end part is advantageously rounded.

According to one embodiment, the cavity comprises at least three successive substantially cylindrical parts starting from the opening, i.e. a first substantially cylindrical part adjacent to the opening having a first diameter, a second substantially cylindrical part adjacent to the first substantially cylindrical part and having a diameter smaller than the diameter of the first substantially cylindrical part, and a third substantially cylindrical part having adjacent to the second substantially cylindrical part and having a diameter smaller than the diameter of the second substantially cylindrical part. Said first and second successive substantially cylindrical parts are joined to each other by an intermediate zone forming a shelf or a step extending between a substantially circular part of an edge of the first substantially cylindrical part and a substantially circular part of an edge of the second substantially cylindrical part, said substantially circular parts of said edges of said first and second substantially cylindrical parts being separated from one another by a distance measured perpendicular to the central axis of the cavity of between 0.03 times and 0.25 times the diameter of the first substantially cylindrical part. Said second and third successive substantially cylindrical parts are joined to each other by an intermediate zone forming a shelf or a step extending between a substantially circular part of an edge of the second substantially cylindrical part and a substantially circular part of an edge of the third substantially cylindrical part, said substantially circular parts of said edges of said second and third substantially cylindrical parts being separated from one another by a distance measured perpendicular to the central axis of the cavity of between 0.03 times and 0.25 times the diameter of the second substantially cylindrical part.

According to a more specifically preferred embodiment, the cavity comprises at least four successive substantially cylindrical parts starting from the opening, i.e. a first substantially cylindrical part adjacent to the opening having a first diameter, a second substantially cylindrical part adjacent to the first substantially cylindrical part and having a diameter smaller than the diameter of the first substantially cylindrical part, a third substantially cylindrical part having adjacent to the second substantially cylindrical part and having a diameter smaller than the diameter of the second substantially cylindrical part, and a fourth part extending between the bottom of the cavity, the fourth part advantageously being substantially cylindrical and having a diameter smaller than the diameter of the third substantially cylindrical part. Said first and second successive substantially cylindrical parts are joined to each other by an intermediate zone forming a shelf or a step extending between a substantially circular part of an edge of the first substantially cylindrical part and a substantially circular part of an edge of the second substantially cylindrical part, said substantially circular parts of said edges of said first and second substantially cylindrical parts being separated from one another by a distance measured perpendicular to the central axis of the cavity of between 0.03 times and 0.25 times the diameter of the first substantially cylindrical part. Said second and third successive substantially cylindrical parts are joined to each other by an intermediate zone forming a shelf or a step extending between a substantially circular part of an edge of the second substantially cylindrical part and a substantially circular part of an edge of the third substantially cylindrical part, said substantially circular parts of said edges of said second and third substantially cylindrical parts being separated from one another by a distance measured perpendicular to the central axis of the cavity of between 0.03 times and 0.25 times the diameter of the second substantially cylindrical part. Said third and fourth successive substantially cylindrical parts are joined to each other by an intermediate zone forming a shelf or a step extending between a substantially circular part of an edge of the third substantially cylindrical part and a substantially circular part of an edge of the fourth substantially cylindrical part, said substantially circular parts of said edges of said third and fourth substantially cylindrical parts being separated from one another by a distance measured perpendicular to the central axis of the cavity of between 0.03 times and 0.25 times the diameter of the third substantially cylindrical part.

According to an advantageous feature, the bottom of the cavity is substantially rounded. Preferably, the radius of curvature of the bottom of the cavity is larger than the radius of curvature of the lower end surface. The thickness of the portion of the structure located between the bottom of the cavity and the end surface is advantageously at least 1 mm, and preferably between 2 and 5 mm.

According to an advantageous detail, the intermediate zone extending between two successive substantially cylindrical parts of the cavity has a curved, advantageously convex, profile.

According to a feature of advantageous embodiments, the cavity is associated with at least one protuberance substantially parallel to the axis of the cavity, said protuberance extending into the cavity and toward its central axis and serving as a guide means for an end of an implant.

Preferably, the cavity is associated with two or three protuberances extending into the cavity and serving as guide means for the placement of the end of an implant into the cavity.

Advantageously, said protuberance(s) do not extend along the entire length of the cavity.

For example, said protuberance(s) extend along only a part or parts of the cavity near the opening.

According to an advantageous feature, the protuberance(s) are contoured so as to have a more tapered shape near the opening of the cavity or at its end facing the opening of the cavity.

According to an advantageous feature, the thickness of the structure at the level of the part adjacent to the opening is smaller than the thickness of the structure at the level of the bottom of the cavity. A larger thickness at the level of the bottom of the cavity or the end of the structure opposite the one provided with the opening enables the formation of a greater bone volume, despite the fact that this portion or end is at a distance from the bone or the bone structure into which an implant must be placed.

The thickness of the structure thus advantageously varies along its length from the opening to the end, progressively increasing with one or more ledges, thus producing an effect of pumping bone cells to the end portion of the structure into which the bottom of the cavity extends.

According to a preferred embodiment, the average thickness (of the lateral wall) of the structure at the level of the opening is greater than 0.2 mm, while the average thickness (of the lateral wall) of the structure at the level of the bottom of the cavity is equal to at least twice, or advantageously at least three times the average thickness (of the lateral wall) of the structure at the level of the opening.

According to an advantageous feature, the substantially cylindrical parts have a length that varies between 0.75 times the average length and 1.25 times the average length, the average length of the substantially cylindrical parts being determined by dividing the total length of the cavity by the number of parts. Advantageously, the part of the substantially cylindrical cavity adjacent to the opening has a length greater than that of the part of the cavity adjacent to the bottom of the cavity.

According to a feature of a preferred embodiment, the structure has a substantially cylindrical outer surface.

Preferably, the cavity of the structure is at least partly formed by mechanical machining, particularly by drilling. According to another advantageous feature, the external shape of the structure is at least partly mechanically machined.

Advantageously, the biomaterial granules are dispersed in a calcium sulfate phase in a substantially homogeneous fashion. The granules are preferably relatively close to each other.

Preferably, the structure contains water, part of which is chemically bonded to the biomaterial granules, the content of water chemically bonded to the biomaterial being between 0.1% by weight and 1% by weight relative to the weight of the biomaterial granules in dry form.

According to an advantageous feature, the biomaterial granules themselves have an apparent density of between 0.5 and 0.6 g/cm³.

According to yet another advantageous feature, the calcium sulfate is essentially in dihydrate form. For example, more than 90% by weight (in particular more than 95% by weight, and more particularly more than 98% by weight) of the calcium sulfate is in dihydrate form.

Advantageously, the structure has a porosity higher than that of a calcium sulfate matrix containing the same quantity of biomaterial and prepared at atmospheric pressure from an aqueous paste of calcium sulfate hemihydrate and biomaterial granules and/or a gas bubble volume that is reduced relative to the gas bubble volume of a calcium sulfate matrix prepared at atmospheric pressure from an aqueous paste of calcium sulfate hemihydrate. This lower porosity and/or lower gas volume present in the calcium sulfate matrix makes it possible to slow the dissolution of the calcium sulfate and thus makes it possible to further increase the growth of the bone-regenerating tissue along the osteointegratable coating.

Preferably, the structure is composed of more than 90% by weight, advantageously more than 95% by weight, and preferably more than 98% by weight of (a) calcium sulfate in dihydrate form, (b) water and/or saline, and (c) biomaterial granules.

The pores of the biomaterial with a diameter of between 50µm and 100µm are advantageously at least partially filled with calcium sulfate. For example, at least 10% (advantageously at least 15%, and preferably at least 20%) of the porous volume formed by the pores of the calcium phosphate biomaterial granules with a diameter of between 50µm and 100µm.

The structure is advantageously placed, prior to use, in a sterile vacuum pack.

Another subject of the invention is an ensemble, particularly a kit, comprising at least one structure according to the invention (having one or more features of the structure of the invention described above in the present specification or in any of the claims) and an implant, advantageously made of titanium, having a body with at least one ledge, said body having a shape adapted to the shape of the cavity of the structure.

Another subject of the invention is a bone reconstruction material essentially comprising pharmaceutical grade calcium sulfate and a slowly or non-resorbable calcium phosphate or apatite biomaterial, said material having one or more features of the bone reconstruction material of a structure according to the invention as defined in the present specification or in any of the claims.

Another subject of the invention is a tool for creating a perforation or cavity in a bone structure, said tool comprising:
- a metal insert having a free end having a cutting edge, a point, an angle or an inclination, and
- a device adapted for generating a sonic or ultrasonic cavitation of the insert and for moving the insert inside the bone structure,
said insert having a channel adapted for supplying a liquid under pressure to the free end having the cutting edge, point, or inclined surface (end). Preferably, the tool includes a means for sealing the bone in the vicinity of the surface through which the insert is introduced and/or for controlling the pressure of the liquid flowing through the hole being formed.

The irrigation system of this tool makes it possible to detach the Schneider membrane before the tool pierces the sinus, thanks to the delivery of a liquid under pressure during the cavitation of the insert inside the bone tissue.

Thus, another subject of the invention is a method for producing a cavity or hole in a bone or bone structure by sonic or ultrasonic cavitation of an insert with a cutting end or edge or an end with a point. This makes it possible to lyse the bone tissue. In addition, such a treatment makes it possible to destroy bacteria and is not traumatic for the surrounding tissues, due to the absence of rotational movement. Better control of the heating of the insert is also possible. Given that there is less heating of the bone during the treatment, it is possible to prevent necrosis of the bone or to strictly limit such necrosis if has begun to form.

Another subject of the invention is a method for producing a cavity or hole in a bone or bone structure in contact with a membrane, particularly a sinus membrane, in which the cavity or hole in the bone or bone structure is pierced by means of an insert or a drill while a liquid under pressure is injected through the free end. The pressure is advantageously capable of passing the liquid through at least a portion of the bone. The passage of liquid through the bone or bone part is improved or better controlled by providing a sealing means between the insert and the bone to be pierced.

Some of the features and details of the invention will emerge from the following description, in which reference is made to the attached drawings.

In these drawings:
- Fig. 1 is a side view of an implant;

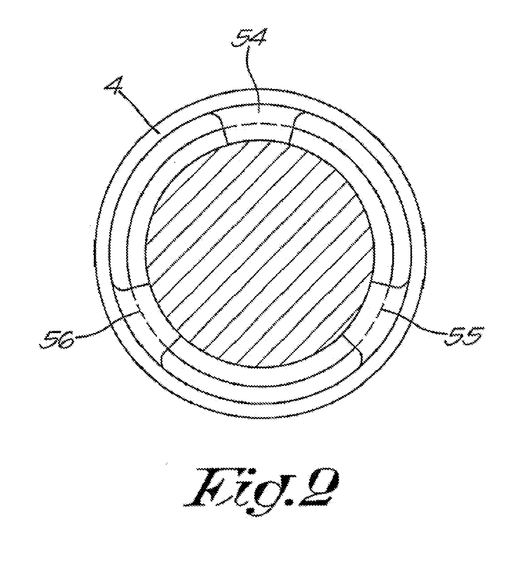

- Fig. 2 is a sectional view of the implant of Fig. 1 along line II-II;

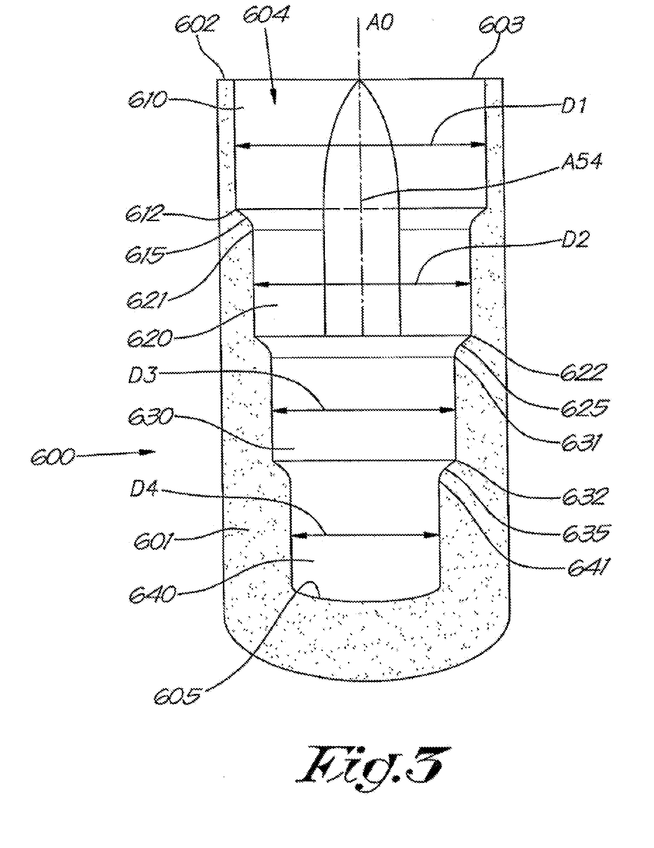

- Fig. 3 is a sectional view of a structure according to the invention adapted for receiving a part of the implant of Fig. 1;

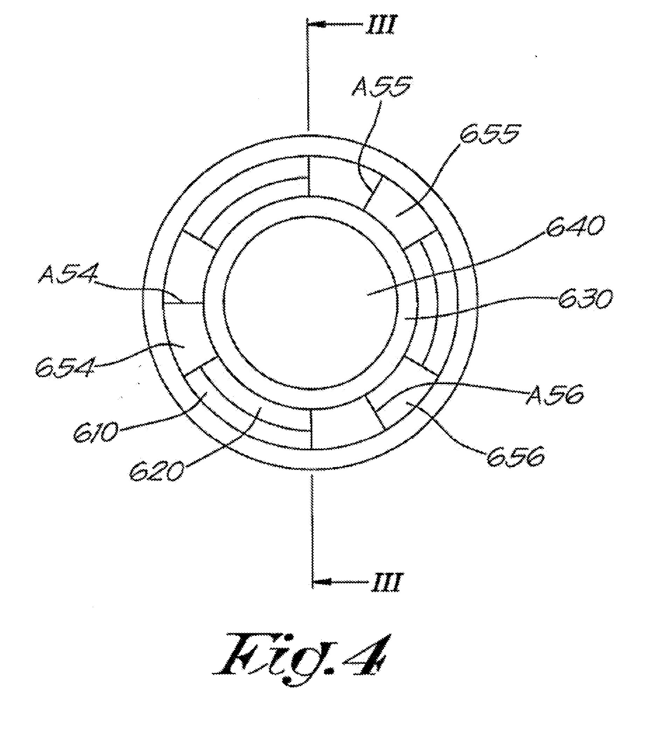

- Fig. 4 is a top view (from the surface provided with the opening) of the structure of Fig. 3;
- Fig. 5 is a side view of a tool for forming a hole by cavitation;
- Fig. 6 is a view of the end of the tool of Fig. 5;
- Fig. 7 is a view of another tool for forming a hole in the sinus;
- Figs. 8A through 8D schematically illustrate steps for the installation of an implant according to the invention;
- Fig. 9 is a schematic view of a tool according to the invention;
- Fig. 10 is an enlarged schematic (SEM) view of a biomaterial agglomerate; and
- Fig. 11 is an infrared spectroscopy (absorbance curve AU/cm⁻¹ of wavelength) of the biomaterial.

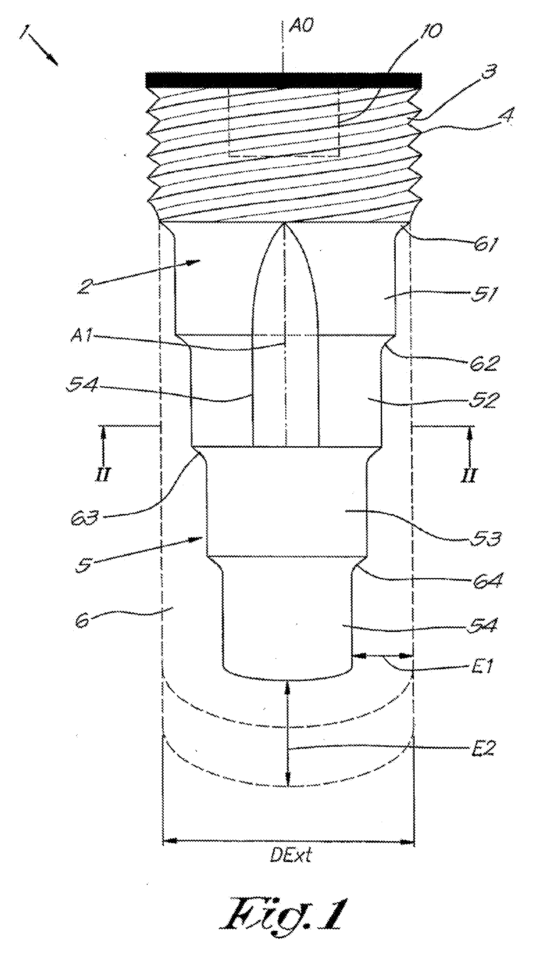

The implant 1 of Fig. 1 comprises a central body 2 made of titanium, an end portion 3 of which has a thread 4 for forming a primary fixation when the implant is placed at least partly inside a cavity of a bone. This central body 2 comprises a portion 5 composed of four substantially cylindrical stages 51, 52, 53, 54 arranged in a pyramid shape relative to each other so as to define a series of ledges, shelves or steps 61, 62, 63, 64. The stage 54 furthest from the threaded portion 3 has the smallest diameter. The diameter of the cylindrical stages decreases the further they are from the threaded portion. The two stages 51, 52 adjacent to the threaded portion 3 have three peripheral channels 70, 71, 72 whose axis A1 is parallel to the central axis A0 of the implant. The difference between the diameter of a first stage and the diameter of a stage directly adjacent to said first stage is substantially constant.

The portion 5 is intended to be inserted into a cavity of an osteointegratable structure 6 whose external diameter DExt is substantially constant and substantially equal to the diameter of the root of the thread 4 of the portion 3. The end of the coating 6 is substantially rounded.

The structure 6 is prepared by mixing calcium sulfate dihydrate with granules of particles of Algipore® (Friadent GmbH) that are smaller than 500µm, said porous calcium phosphate granules themselves having a specific surface area of about 15m²/g and a macroporosity constituted by pores or cells with an average diameter of between 50µm and 100µm. The apparent density of the porous particles was about 0.55 g/ml.

Pharmaceutical grade calcium sulfate dihydrate having in dry form a magnesium content of less than 2% by weight (advantageously less than 1% by weight) was mixed with water to form a paste. Calcium phosphate particles with a grain size fraction of less than 500µm were added to this paste.

This mixing operation was performed under reduced pressure, or advantageously under vacuum or under a certain vacuum.

The pressure was lower than the atmospheric pressure, for example less than 0.5 10⁵ Pa (0.5 bar), but advantageously even lower (less than 0.1 10⁵ Pa, and in particular less than 0.03 10⁵ Pa) during the ingredient mixing operation. This makes it possible to limit the formation of bubbles in the calcium sulfate matrix.

Advantageously, the porous calcium phosphate particles are vacuum-processed prior to being incorporated into the calcium sulfate + water mixture.

It was observed that when operating at low pressure, particularly under vacuum, it was possible to reduce the porosity of the calcium sulfate dihydrate. It was also observed that some of the pores of the porous calcium phosphate particles contained calcium sulfate.

The structure 6 is described below.

As may be seen, the structure 6 is independent of the implant prior to osteointegration. This allows easier placement of the various elements after they are quality-controlled. Moreover, the formation of bone between the structure and the implant will also allow a better fixation of the implant into a larger bone volume, through the formation of bone between the structure and the implant.

The structure 6 is in the shape of a tubular sleeve 600, one end of which is closed. The structure comprises an osteogenic body 601 made from a bone reconstruction material essentially comprising pharmaceutical grade calcium sulfate and a slowly or non-resorbable calcium phosphate or apatite biomaterial in the form of porous granules having an average grain size by weight of between 250 and 3000µm, and in particular between 300µm and 1000µm. Said biomaterial granules themselves having a specific surface area of between 10 and 50 m²/g (in particular between 10 m²/g and 20 m²/g), a macroporosity formed of pores with an average diameter (in porous volume) of between 50 and 100µm. Said osteogenic body having a substantially flat end or surface 602 with an opening 603 extended by a central cavity 604 with a central axis A0 and adapted for receiving at least one end of an implant of the type shown in Fig. 1.

The cavity 604 comprises at least four successive substantially cylindrical parts starting from the opening, i.e. a first substantially cylindrical part 610 adjacent to the opening 603 having a first diameter D1, a second substantially cylindrical part 620 adjacent to the first substantially cylindrical part 610 and having a diameter D2 smaller than the diameter D 1 of the first substantially cylindrical part, a third substantially cylindrical part 630 having adjacent to the second substantially cylindrical part 620 and having a diameter D3 smaller than the diameter D2 of the second substantially cylindrical part, and a fourth part 640 extending between the bottom 605 of the cavity 604 and the third part 630, the fourth part 640 advantageously being substantially cylindrical and having a diameter D4 smaller than the diameter D3 of the third substantially cylindrical part.

Said first and second successive substantially cylindrical parts 610, 620 are joined to each other by an intermediate zone 615 forming a shelf, a step, or a ledge extending between a substantially circular part of an edge 612 of the first substantially cylindrical part 610 and a substantially circular part of an edge 621 of the second substantially cylindrical part 620. Said substantially circular parts of said edges 612, 621 of said first and second substantially cylindrical parts 610, 620 are separated from one another by a distance (a distance equal to D1 - D2 divided by two) measured perpendicular to the central axis A0 of the cavity 604 of between 0.03 times and 0.25 times the diameter D1 of the first substantially cylindrical part 610. Said second and third successive substantially cylindrical parts 620, 630 are joined to each other by an intermediate zone 625 forming a shelf, a step, or a ledge extending between a substantially circular part of an edge 622 of the second substantially cylindrical part 620 and a substantially circular part of an edge 631 of the third substantially cylindrical part 630. Said substantially circular parts of said edges 622, 631 of said second and third substantially cylindrical parts 620, 630 are separated from one another by a distance (a distance equal to D2 - D3 divided by two) measured perpendicular to the central axis A0 of the cavity 604 of between 0.03 times and 0.25 times the diameter D2 of the second substantially cylindrical part 620. Said third and fourth successive substantially cylindrical parts 630, 640 are joined to each other by an intermediate zone 635 forming a shelf, a step, or a ledge extending between a substantially circular part of an edge 632 of the third substantially cylindrical part 630 and a substantially circular part of an edge 641 of the fourth substantially cylindrical part 640, said substantially circular parts of said edges 632, 641 of said third and fourth substantially cylindrical parts 630, 640 being separated from one another by a distance (a distance equal to D3 - D4 divided by 2) measured perpendicular to the central axis A0 of the cavity 604 of between 0.03 times and 0.25 times the diameter D3 of the third substantially cylindrical part 630.

The bottom 605 of the cavity 604 is curved (a substantially rounded shape), the center point of the bottom being the furthest from the opening 603.

The intermediate zones 615, 625, 635 extending between two successive substantially cylindrical parts of the cavity 604 have a curved, advantageously convex, profile. Thus, the surface of these intermediate zones is angled toward the cavity relative to a line extending between a point of a substantially circular part of an edge of a first substantially cylindrical part and between another point of a substantially circular part of an edge of another substantially cylindrical part adjacent or successive to the first.

The cavity 604 is associated with three protuberances substantially parallel to the axis A0 of the cavity 604, said protuberances extending into the cavity and toward its central axis and serving as guide means for an end of an implant.

The cavity 604 is associated with two or three protuberances extending into the cavity and serving as guide means for the placement of the end of an implant (see Fig. 1) into the cavity 604. These protuberances 654, 655, 656 each have a tapered profile with a plane of symmetry. The planes of symmetry A54, A55, A56 are disposed 120° apart from each other relative to the axis A0, said planes intersecting along the axis A0.

The end of the protuberances 654, 655, 656 facing the opening 603 forms a point or a part of small width so as to promote the sliding of the channels 70, 71, 72 and the positioning of the implant 1 relative to the cavity. The free edge of one or more channels 70, 71, 72 is capable of sliding over the contoured lateral wall of a protuberance.

When the structure has such protuberances 654, 655, 656, the structure and the implant are joined together prior to fixing the implant with the threaded portion to a bone or bone structure.

The thickness of the structure at the level of the part adjacent to the opening 603 is smaller than the thickness of the structure at the level of the bottom 605 of the cavity 604. This thickness increases with the jumps in thickness.

The average thickness of the structure at the level of the opening 603 is greater than 0.2 mm, while the average thickness of the structure at the level of the bottom 605 of the cavity is equal to at least twice, or advantageously at least three times the average thickness of the structure at the level of the opening.

The substantially cylindrical parts 610, 620, 630, 640 have a substantially equal length L.

The structure is advantageously prepared from a block made of bone-forming material. This block is then machined to form a cavity formed of several parts, and to shape the external wall of the structure. This makes it possible to give the structure surfaces having a porosity or pores that correspond to the inner pores of the block prior to its being shaped. This machining is, for example, a mechanical machining, in particular by drilling.

The structure 6 advantageously comprises biomaterial granules dispersed in a calcium sulfate phase in a substantially homogeneous fashion. It contains water, part of which is chemically bonded to the biomaterial granules, the content of water (of constitution) chemically bonded to the biomaterial being between 0.1 % by weight and 1% by weight relative to the weight of the biomaterial granules.

The biomaterial granules themselves have an apparent density of between 0.5 and 0.6 g/cm³_{.}

The calcium sulfate is essentially in dihydrate form.

The structure has a porosity higher than that of a calcium sulfate matrix containing the same quantity of biomaterial and prepared at atmospheric pressure from an aqueous paste of calcium sulfate hemihydrate and biomaterial granules.

In particular, the structure is composed of more than 90% by weight, advantageously more than 95% by weight, and preferably more than 98% by weight of calcium sulfate in dihydrate form, water and/or saline, and biomaterial granules.

Various mixtures were thus prepared, the ratio by weight of calcium sulfate dihydrate to calcium phosphate particles of the mixtures being as follows:
Mixture 1: ratio by weight in dry form
   calcium sulfate dihydrate to calcium phosphate granules of 3
Mixture 2: ratio by weight in dry form
   calcium sulfate dihydrate to calcium phosphate granules of 2.5
Mixture 3: ratio by weight in dry form
   calcium sulfate dihydrate to calcium phosphate granules of 2
Mixture 4: ratio by weight in dry form
   calcium sulfate dihydrate to calcium phosphate granules of 1.5
Mixture 5: ratio by weight in dry form
   calcium sulfate dihydrate to calcium phosphate granules of 1

The mixtures with a ratio by weight in dry form of calcium sulfate dihydrate to porous calcium phosphate granules of between 1 and 2.5 are preferred.

The mixture is advantageously prepared under vacuum (partial vacuum or at reduced pressure) and is poured under vacuum into a mold. The mixture may contain one or more other agents, surface-active agents, polymers, fillers, etc., the content of other agents, additives and fillers advantageously being less than 10% by weight of the dry structure (dried at 80°C to eliminate all free water), and in particular less than 5% by weight of the structure. Such agents are, for example, polysaccharide agents capable of forming a gel, hyaluronic acid, antibiotics, growth factors, etc.

The threaded portion 3 of the implant 1 has a fastening system 10, for example for a tooth, a prosthesis, a joint, etc. This fastening system 10 is for example a recess with a threading formed inside the threaded head 3 for the fixation of a tooth, a prosthesis, a joint, etc.

For the placement of the implant into the upper maxilla, it is advantageous to preserve the integrity of the sinus membrane.

This is done using a tool that makes it possible to simultaneously perforate the bone structure, while making it possible, when the tool or the insert is close to the membrane (while still being inside the bone) to perform a progressive detachment of the sinus membrane.

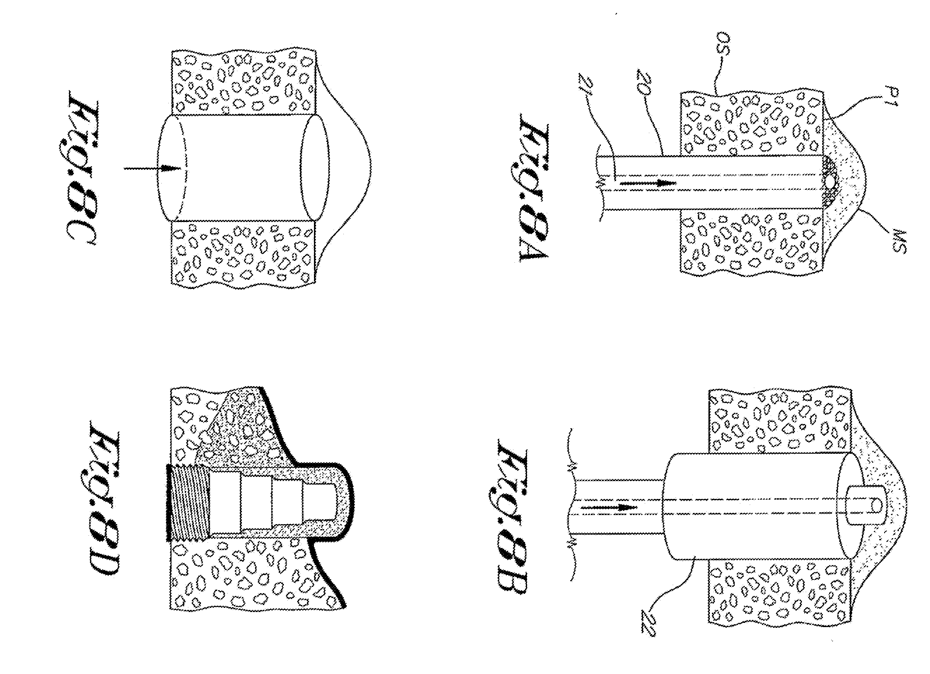

In a first method (see Figs. 8), the bone structure (OS) is drilled with a guide drill 20 having a central channel 21 for supplying a liquid under pressure. Bone being a porous structure, the liquid under pressure in the channel flows partially into the bone. However, as long as the free end of the guide drill is at a distance from the inner bone wall P1, the pressure of the liquid inside the channel is substantially constant. When the end of the drill is adjacent to the inner wall PI, the liquid under pressure flows easily into the bone toward the sinus membrane MS, thus making it possible to perform a progressive detachment of the membrane before the guide drill 20 pierces the bone structure OS.

The pressurization of the liquid may or may not be continuous, for example, occurring in successive stages. For example, the liquid is subjected to a pressurization cycle comprising high pressure stages and low- or average- and/or no-pressure stages.

This drop in pressure also makes it possible to warn the practitioner that the bone structure OS will soon be pierced. This signal is also advantageously used to trigger an injection or control an injection of a precise volume of liquid in order to ensure an adequate detachment of the sinus membrane. For example, once the pressure drop signal is detected, a volume of 5 ml of aseptic liquid (water) is injected. When the 5 ml of liquid are injected, the rotation of the guide drill is interrupted.

In order to ensure that the liquid under pressure actually passes through the bone, it is advantageous for the tool to be equipped with a means for sealing the bone in the vicinity of the surface through which the insert is introduced and/or checking the pressure of the liquid flowing into the hole being formed (for example for cooling the insert and the inside walls of the hole as it is pierced).

A hole of larger diameter is then drilled along the guide drill by means of one or more drills 22. During the complementary drilling along the guide drill to increase the diameter of the hole, it may be appropriate to check the pressure of liquid in the channel 21 of the guide drill. For example, as soon as this pressure falls below a given level, a control device re-supplies water under pressure to ensure that the sinus membrane is still properly detached and separated from the bone structure near the location of the hole to be drilled.

When the hole has a diameter that substantially corresponds to the diameter of the threaded portion of the implant, the drill and the guide drill are withdrawn.

A grafting composition is then injected through the hole and the implant is then inserted. Thanks to the threading of the portion 3, it is possible to obtain a primary stabilization of the implant on the bone structure before the secondary stabilization is produced, thanks to the transformation of the coating into a layer of bone due to a fast colonization of the coating by the patient's bone cells.

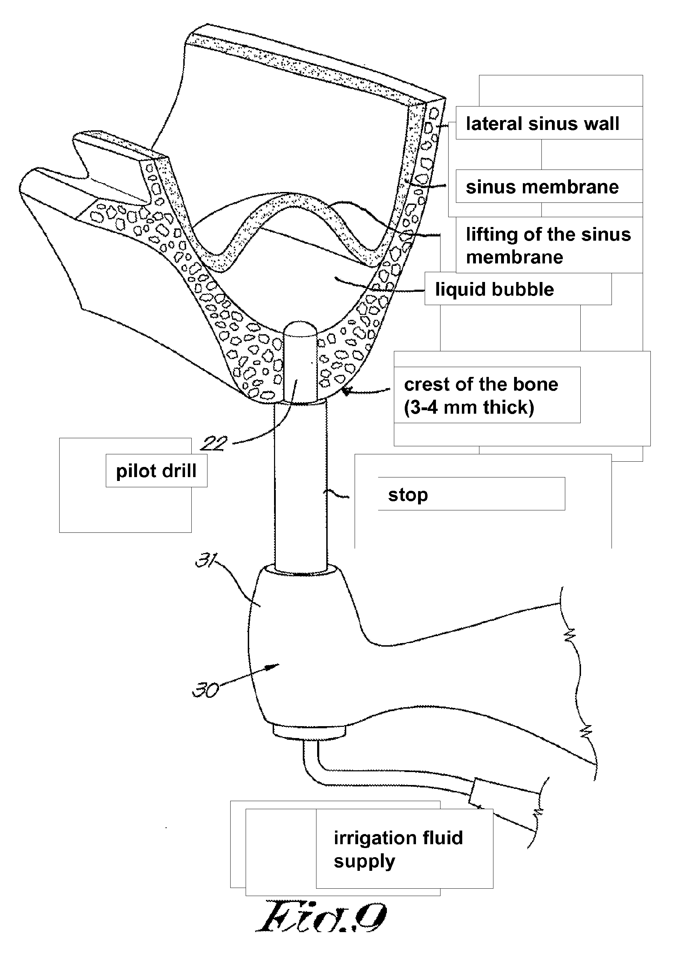

Fig. 9 is a schematic perspective view of a perforation device that carries a drill 22.

This device comprises a support 30 whose head 31 is equipped with a means for driving the drill 22 in rotation. The head 31 is associated with a stop 32 whose position is advantageously adaptable as a function of the length of the movement for piercing the bone with the guide drill, a length defined by a length that substantially corresponds to the length at which a drop in pressure is determined.

Instead of using one or more drills to pierce the bone structure or form a hole for receiving the implant, a tool comprising a metal insert 40 having one or more cutting edges 41 with an end head covered with diamonds 42 was used, along with a device generating an ultrasonic or sonic (particularly ultrasonic) cavitation of the insert inside the bone structure while advancing the insert into the bone structure.

The insert has an inner channel 43 adapted for supplying a liquid under pressure.

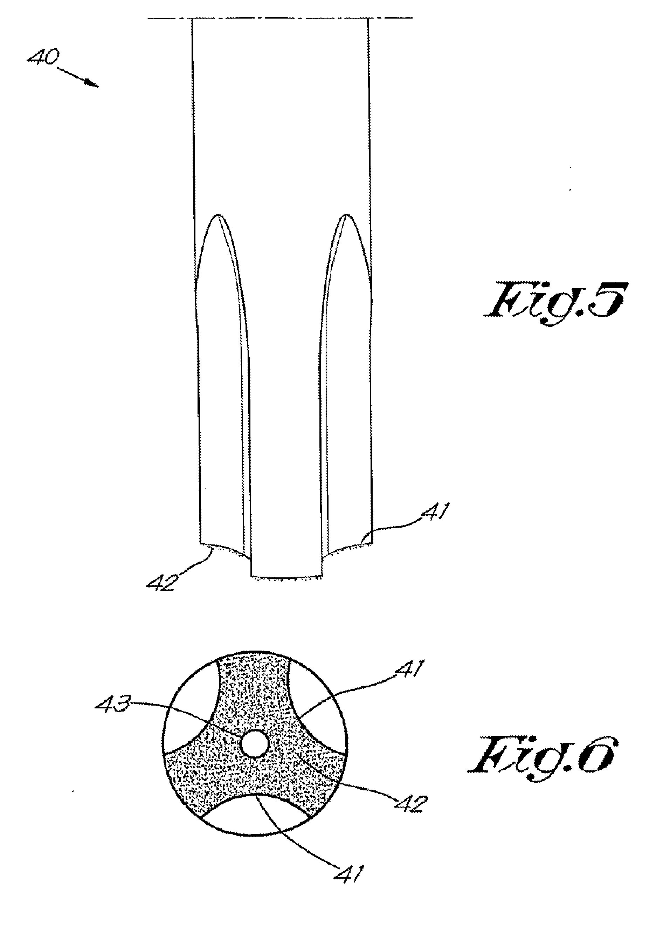

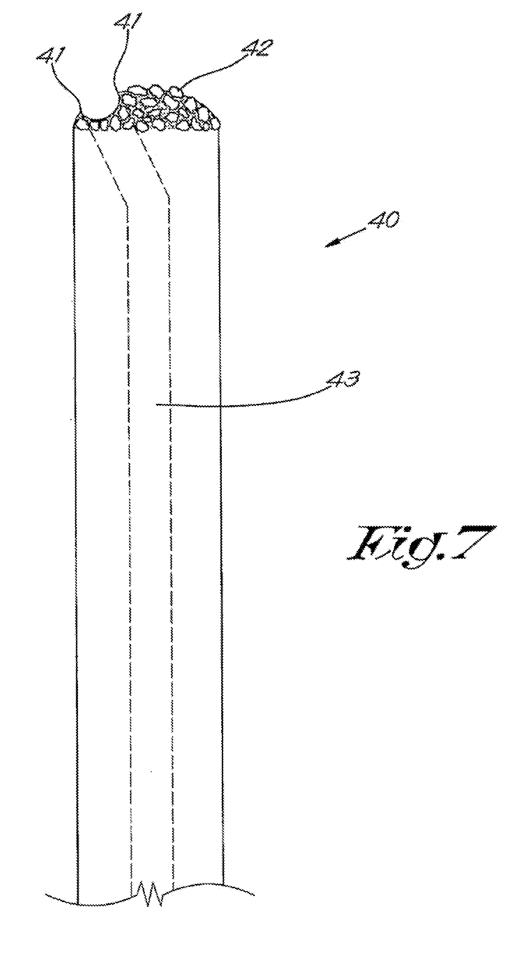

Fig. 7 is a view of another insert similar to that of Fig. 5. The total length of the insert 40 is for example less than 35 mm.

In order to ensure that the liquid under pressure actually passes through the bone, it is advantageous for the tool to be equipped with a means for sealing the bone in the vicinity of the surface through which the insert is introduced and/or for controlling the pressure of the liquid flowing into the hole being formed (for example, for cooling the insert and the inside walls of the hole as it is pierced.)

The insert has elasticity both in the direction of the advance of the insert into the bone and in the opposite direction. The insert will be made to vibrate in the direction of the advance of the insert into the bone, creating sorts of cavitation bubbles.

During the operation for piercing a hole by ultrasonic cavitation, a liquid under pressure is supplied through the inner channel of the insert. This liquid is for example water, physiological saline, an isotonic saline solution, or a solution of water containing from 0.5% by weight to 3% by weight of NaCl, for example from 0.7 to 1.5% by weight of NaCl. This liquid is pressurized, either continuously or otherwise.

When the liquid is pressurized, part of this liquid passes into the porous structure of the bone, making its way through the bone mass of type II, II and IV. As long as the free end of the insert is far from the sinus membrane, the pressure of the liquid in the channel is high.

Once the end of the insert is adjacent to the wall of the bone in contact with the Schneider membrane (sinus membrane), the liquid will pass through the bone to detach the Schneider membrane. A pocket of liquid will then be formed between the sinus floor and the membrane.

It was observed that the creation of a bone channel by ultrasonic cavitation made it possible to lyse bone tissue, for example of type II, III and IV. Such mechanically induced lysis is surgically useful in creating a channel in the bone in a way that is non-traumatic for the surrounding tissues, but destroys any bacteria in them.

The heating of the insert can be better controlled, for example by means of an irrigation fluid, which makes it possible to prevent necroses at the level of the hole, but has proven especially useful in producing better osteointegration, particularly when using the implant according to the invention.

Using a channel created by ultrasonic cavitation and an implant according to the invention (an implant carrying a ball-and-socket joint or having a device for attaching a ball-and-socket joint), it is possible to obtain osteointegration of orthopedic prostheses. The implant according to the invention is symbiotic, the osteointegration being osteosymbiotic.

In the case of bone of type I, for example tumorous bone, it was observed that the implant according to the invention, thanks to its particular coating, makes it possible to compensate for inertia in the osteogenerative biodynamics. This will make it possible to provide total stability of the implant after osteointegration. In order to provide a primary stabilization, the implant is fixed to the bone by means of the threading of the top part of the implant. After this primary stability, the creation of bone tissue on the surfaces of the structure and inside the structure will form a layer of bone around the titanium body, which will then ensure secondary stability.

The implant may directly comprise an articular shelf or step, a femur head, etc., but is advantageously of the type having a means for enabling, advantageously after osteointegration of the implant, the placement and fixation of a shelf or step, a joint, a femur head, etc. The structure according to the invention can be used in the bones of the elbow, the shoulder, the knee, the hip, i.e. all of the long bones in general.

The structure according to the invention, and thus the bone reconstruction material has proven to have a catalytic effect on bone formation, particularly in bones with a low propensity for osteointegration.

One biomaterial that is particularly useful for the structure according to the invention is in the form of porous granules whose composition essentially corresponds to carbonated hydroxyapatite with traces of protein-type organic materials. The water of constitution content is on the order of 0.2 to 1% by weight, the organic matter content is between 2% by weight and 3% by weight, while the mineral phase constitutes the rest. The calcium carbonate content is on the order of 2 to 3% by weight.

Fig. 9 shows the X-ray diffraction graph of the preferred biomaterial, measured at or converted to the wavelength of 1.54 Å. As is clear from this graph, the intensity is low, demonstrating a moderate hydroxyapatite crystalline phase. This XR diffraction curve is very similar to that of a sterilized bone powder of human origin.

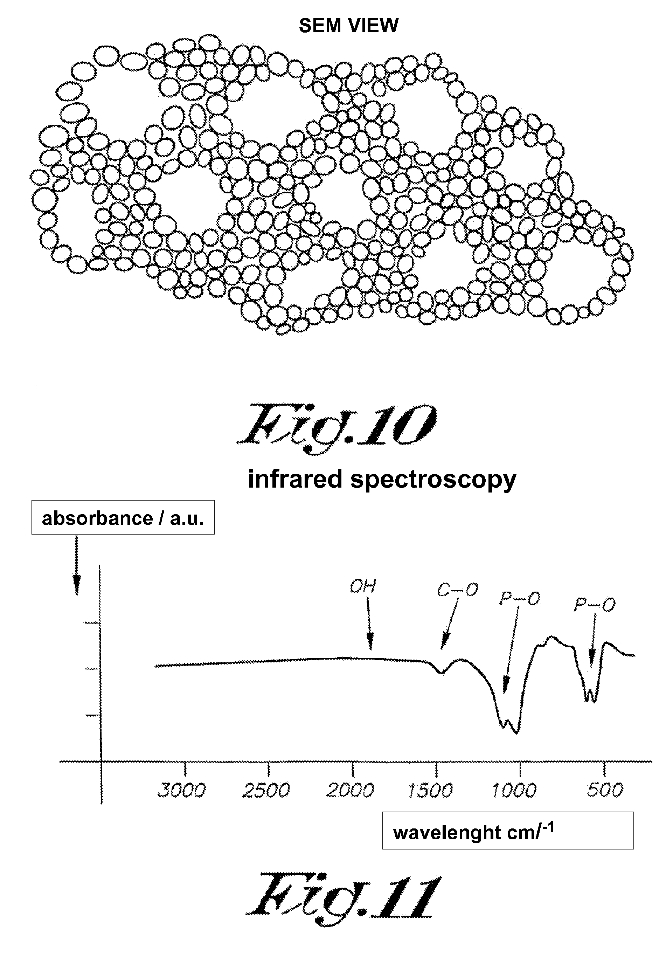

Fig. 10 is a schematic SEM image of a preferred biomaterial.

## Claims

1. Tool for creating a perforation or cavity in a bone or bone structure in contact with a membrane, said tool comprising:
- a drill (20, 22) having a free end and a channel (21) adapted for supplying a liquid under pressure to the free end;
- means for providing said liquid under pressure such that said liquid flows partially into and through the bone or bone structure toward the membrane while piercing the bone or bone structure with the drill, allowing to perform a progressive detachment of the membrane when the drill is close to the membrane while still being inside the bone or bone structure.

2. Tool according to the preceding claim wherein said membrane is a sinus membrane (MS).

3. Tool according to any one of the preceding claims wherein said bone is an upper maxilla.

4. Tool according to any one of the preceding claims further comprising means for detecting a drop in pressure of said liquid.

5. Tool according to the preceding claim further comprising means for triggering an injection or controlling an injection of a precise volume of liquid for ensuring an adequate detachment of the membrane.

6. Tool according to any one of the preceding claims further comprising means for pressurizing said liquid continuously or non-continuously.

7. Tool according to any one of the preceding claims further comprising a sealing means between the drill and the bone or bone structure to be pierced.

8. Tool according to any one of the preceding claims further comprising a stop (32).

9. Method for creating a perforation or cavity in a bone or bone structure in contact with a membrane, the method comprising the steps of:
- piercing said bone or bone structure by a drill (20, 22);
- providing a liquid under pressure at a free end and through a channel (21) of said drill such that said liquid flows partially into and through the bone or bone structure toward the membrane while piercing the bone or bone structure with the drill, allowing to perform a progressive detachment of the membrane when the drill is close to the membrane while still being inside the bone or bone structure.

10. Method according to claim 9 wherein said membrane is a sinus membrane (MS).

11. Method according to claim 9 or claim 10 wherein said bone is an upper maxilla.

12. Method according to any one of claims 9 to 11 further comprising detecting a drop in pressure of said liquid.

13. Method according to claim 12 further comprising triggering an injection or controlling an injection of a precise volume of liquid for ensuring an adequate detachment of the membrane.

14. Method according to claim 13 further comprising interrupting rotation of said drill (20, 22) when injecting said precise volume of liquid.

15. Method according to any one of claims 9 to 14 further comprising pressurizing said liquid continuously or non-continuously.
